# EUROPEAN PATENT APPLICATION

(11) **EP 4 311 544 A1**
(43) Date of publication of application: **31.01.2024**
(21) Application number: 22382739.5
(22) Date of filing: 29.07.2022
(51) Int. Cl.: A61K 31/045, A61K 31/047, A61K 31/12, A61P 17/02, A61K 8/34, A61K 8/35, A61Q 17/04, A61K 9/00

(54) **COMPOUNDS USEFUL AGAINST JELLYFISH STINGS**

(71) Applicant: Isdin, S. A., 08019 Barcelona (ES)
(72) Inventor: TRULLAS CABANAS, Carlos Ramon, 08019 BARCELONA (ES); JOURDAN, Eric Michel René, 08019 BARCELONA (ES); BALLESTEROS MASCARELL, Ainara, 08003 BARCELONA (ES); GILI SARDÀ, Jose Maria, 08003 BARCELONA (ES)
(74) Representative: ZBM Patents - Zea, Barlocci & Markvardsen

(57) **Abstract**

It relates to the use of an active ingredient selected from hydroxy acetophenone, a topically acceptable salt thereof, a combination of hydroxy acetophenone with a glycol, and a combination of a topically acceptable salt of hydroxy acetophenone with a glycol, for avoiding the sting of a poisonous organism that contain cnidocytes. This active ingredient may be combined with a second active ingredient selected from cis-4-tert-butylcyclohexanol, trans-4-tert-butilcyclohexanol, a mixture of cis-4-tert-butylcyclohexanol and trans-4-tert-butilcyclohexanol, and a topically acceptable salt of any of them. It also relates to a topical composition comprising an effective amount for avoiding the sting of a poisonous organism of any of these active ingredients or combinations of them, together with one or more appropriate topical cosmetically acceptable excipients or carriers.

## Description

The present invention relates to the use of compounds for inhibiting nematocysts discharge of cnidarian organisms, in particular jellyfish, as well as, to compositions comprising them.

### Background Art

Jellyfish are found all over the world, from surface waters to the deep sea and a possible increase of jellyfish blooms has been reported in some areas of the world.

Like all cnidarians, they share the same fundamental stinging mechanism involving injection of prey and predator with venom-filled stinging cells called cnidocytes. These cells contain an organelle called nematocyst that work like tiny harpoons by physic-chemical stimuli. The nematocyst capsule stores a large concentration of calcium ions, which are released from the capsule into the cytoplasm of the cnidocyte when the trigger is activated. This causes a large concentration gradient of calcium across the cnidocyte plasma membrane. The resulting osmotic pressure causes a rapid influx of water into the cell. This increase in water volume in the cytoplasm forces the coiled cnidae tubule to eject rapidly. Prior to discharge the coiled cnidae tubule exists inside the cell in an "inside out" condition. The back pressure resulting from the influx of water into the cnidocyte together with the opening of the capsule tip structure or operculum, triggers the forceful eversion of the cnidae tubule causing it to right itself as it comes rushing out of the cell with enough force to impale a prey organism.

Although jellyfish use cnidocytes mainly for prey capture and defense against predators, 150 million jellyfish stings are reported worldwide annually in humans with effects ranging from mild discomfort to serious injury or even life-threatening, being one of the most common reasons for seeking first-aid assistance from lifeguards during bathing seasons.

The jellyfish species that inhabit the Mediterranean coastal water are not lethal, but their sting can cause severe pain and over the last years it has become a more common concern for swimmers. Due to its wide distribution, abundance, and the severity of its sting, *Pelagia noctiluca* has become the most important jellyfish of the Mediterranean. As such, this jellyfish is responsible for a high number of cases requiring assistance from rescue lifeguards and has been classified as a highly venomous species due to the severity of its sting, which generally causes immediate pain, urticaria, edema, a burning sensation and formation of vesicles, papules and/or scabs.

The first-aid protocols for jellyfish stings define a set of actions that allow immediate assistance to those stung by jellyfish. In the sting management scenario, removing residual cnidocytes with a safe rinse solution and relieving pain are key actions for proper management. Over the years, several different rinse solutions have been proposed. Some treatments are based on thoroughly rinse the affected area with vinegar or with a commercial spray, if available, and carefully remove the tentacles. However, the effectiveness of these remedies is often unsatisfactory.

A Ballesteros et al in "Differing Effects of Vinegar on Pelagia noctiluca (Cnidaria: scyphozoa) and Carybdea marsupialis (Cnidaria: Cubozoa) sting. Implications for First Aid Protocols", Toxins, 2021, vol. 13, p.509 compared the effect of vinegar and seawater on the nematocysts discharge process in two species representative of the Mediterranean region and concluded that vinegar in P. *noctiluca* produced nematocysts discharge *per se* but inhibited nematocysts discharge from *Carybdea marsupialis.* Whereas in case of a *cubozoan C. marsupialis* sting, the inhibitory effect of vinegar makes it the ideal rinse solution, in case of a scyphozoan P. *noctiluca* sting, vinegar application may be counterproductive, worsening the pain and discomfort of the stung area.

Several prevention strategies to avoid the sting of cnidarians have been disclosed.

EP2380577A1 discloses the use of a combination of lanthanum and zinc cations for avoiding the sting of poisonous organisms that contain cnidocytes, such as cnidarians and myxozoans.

EP2363135A1 discloses the use of a topical composition comprising an effective amount of at least one plant extract, together with appropriate topical pharmaceutically or cosmetically acceptable carriers and/or excipients, for avoiding the sting of poisonous organisms that contain cnidocytes such as P. *noctiluca.* It further discloses a combination for simultaneous, or sequential administration, of at least one plant extract, and an external source of a cation metal selected from the group consisting of lanthanum, gadolinium, zinc, calcium, sodium, potassium, magnesium, and titanium.

Salleo et al in J. exp. Biol., 1994, vol. 187, pp. 201-206 discloses the use of Gd³⁺ and La³⁺ in preventing stings from harmful Cnidaria.

Finally, WO 98/53807A1 discloses compositions comprising an effective amount of antihistamines and/or an effective amount of at least one metal cation (Ca, Mg, Na, Mn, Co, K and Fe) for inhibiting cnidocyst or polar capsule discharge from several organisms including from P. *noctiluca.*

Despite what is known in the art, due to the enormous health impact of jellyfish stings in coastal areas, identifying solutions to effectively inhibit nematocysts discharge if still of great interest.

### Summary of Invention

The present inventors have found that hydroxy acetophenone (HAP), its topically acceptable salts thereof, as well as a combination of hydroxy acetophenone or its topically acceptable salts with a glycol, inhibit the nematocysts discharge and, therefore, may be useful for avoiding the sting of poisonous organisms that contain cnidocytes, in particular of jellyfish such as P. *noctiluca.*

Thus, a first aspect of the present invention relates to the use of an active ingredient selected from the group consisting of hydroxy acetophenone, a topically acceptable salt thereof, a combination of hydroxy acetophenone with a glycol, and a combination of a topically acceptable salt of hydroxy acetophenone with a glycol, for avoiding the sting of a poisonous organism that contain cnidocytes. This can be achieved by inhibiting nematocysts discharge of poisonous organism that contain cnidocytes. It means that they are suitable for reducing, preferably, stopping nematocysts discharge.

A second aspect of the present invention relates to a topical composition comprising an effective amount for avoiding the sting of a poisonous organism of an active ingredient selected from the group consisting of hydroxy acetophenone, a topically acceptable salt thereof, a combination of hydroxy acetophenone with a glycol, and a combination of a topically acceptable salt of hydroxy acetophenone with a glycol, together with one or more appropriate topical acceptable excipients or carriers. The topical composition is suitable for inhibiting nematocysts discharge of a poisonous organism, in particular, is suitable for reducing, preferably stopping, nematocysts discharge.

### Detailed description of the invention

### Definitions

The terms "nematocyst" is used in the conventional meaning to refer to the subcellular stinging structures found in the cnidocytes. They are capable of penetrating, anchoring, and injecting a toxin into a target subject.

The term "topical composition" as used herein refers to a mixture of an active ingredient, with other chemical components, such as physiologically or topically acceptable carriers and/or excipients, which is appropriate to be applied on the surface of the skin and mucosal cells and tissues. The purpose of the topical composition is to facilitate the application of the active compounds.

The term "topically acceptable salt" as used herein is intended to mean any salt formed from topically acceptable non-toxic bases including inorganic or organic bases. There is no limitation regarding the salts, except that if used for topical purposes, they must be topically acceptable.

The expression "effective amount against the sting of a poisonous organism that contain cnidocytes" or "for avoiding the sting of a poisonous organism that contain cnidocytes" or "for inhibiting the nematocysts discharge" as used herein, refers to an amount of the compound being administered which has the effect of inhibiting, which means reducing to some extent, preferably stopping, nematocysts discharge. The particular dose of compound to be applied according to this invention will of course be determined by the particular circumstances surrounding the case, including the compound administered, alone or in combination, the type of formulation, and the similar considerations.

The expression "topically acceptable excipients or carriers" refers to topically acceptable materials, compositions, or vehicles that can be for instance cosmetically acceptable, pharmaceutically acceptable, or dermatological acceptable excipients or carriers. Each component must be topically acceptable in the sense of being compatible with the other ingredients of the composition. It must also be suitable for use in contact with the skin of humans and animals without excessive toxicity, irritation, allergic response, immunogenicity or other problems or complications commensurate with a reasonable benefit/risk ratio.

As mentioned above it is part of the invention the use of an active ingredient selected from the group consisting of hydroxy acetophenone, a topically acceptable salt thereof, a combination of hydroxy acetophenone with a glycol, and a combination of a topically acceptable salt of hydroxy acetophenone with a glycol, for avoiding the sting of a poisonous organism that contain cnidocytes. In a particular embodiment, this is achieved by inhibiting nematocysts discharge of a poisonous organism that contain cnidocytes.

Examples of such organisms are *Hydrozoa, Anthozoa, Myxozoa and Schyphozoa, in particular, Aurelia spp., Pelagia sp., Chrysaora spp., Anthoplaura spp, Rhopilema spp., Physalia spp., Cyanea psp., Linuche spp., Catostylus spp, Carybdea spp., Chironex spp., Stomolophus spp., Rhiozostom aspp, and Corinactis spp.*

In a particular embodiment of the use according to the invention, the poisonous organism is a jellyfish. In another particular embodiment, the jellyfish Is a P. *noctiluca.*

In another particular embodiment of the use according to the invention, the hydroxy acetophenone is selected from the group consisting of 2-hdyroxyacetophenone, 3-hydroxyacetophenone, 4-hdyroxyacetophenone, and mixtures thereof.

In another particular embodiment, hydroxy acetophenone in free form is used.

In another particular embodiment of the use according to the invention, the glycol is selected from the group consisting of butylene glycol, propylene glycol, and mixtures thereof. In a preferred embodiment the glycol is butylene glycol.

In another particular embodiment, the glycol is pentylene glycol.

In a preferred embodiment of the use according to the invention, a combination of hydroxy acetophenone or a topically acceptable salt thereof, with butylene glycol is used.

In another particular embodiment a combination of hydroxy acetophenone in free form with butylene glycol is used.

The present inventors have also shown that trans-4-tert-butilcyclohexanol, a mixture of cis-4-tert-butylcyclohexanol and trans-4-tert-butilcyclohexanol, topically acceptable salts of any of them, and a combination of any of them with a glycol, inhibit the discharge of nematocysts of poisonous organism that contain cnidocytes, in particular of jellyfish such as P. *noctiluca.* It means that they are suitable for reducing, preferably, stopping nematocysts discharge. Thus, these compounds can be used for avoiding the sting of poisonous organisms. Therefore, the combined use of: a) a first active ingredient which is hydroxy acetophenone or a topically acceptable salt thereof; b) a second active ingredient which is selected from the group consisting of cis-4-tert-butylcyclohexanol, trans-4-tert-butylcyclohexanol, a mixture of cis-4-tert-butylcyclohexanol and trans-4-tert-butylcyclohexanol, and a topically acceptable salt of any of them; for avoiding the sting of a poisonous organism that contain cnidocytes, in particular of jellyfish such as P. *noctiluca,* also forms part of the invention. In a particular embodiment, this is achieved by inhibiting nematocysts discharge of a poisonous organism that contain cnidocytes.

In a particular embodiment of the previous use, it comprises using a combination comprising: a) a first active ingredient which is hydroxy acetophenone or a topically acceptable salt thereof; b) a second active ingredient which is selected from the group consisting of cis-4-tert-butylcyclohexanol, trans-4-tert-butylcyclohexanol, a mixture of cis-4-tert-butylcyclohexanol and trans-4-tert-butylcyclohexanol, and a topically acceptable salt of any of them; and c) a glycol.

Besides, it is considered part of the invention the use of an active ingredient selected from cis-4-tert-butylcyclohexanol, trans-4-tert-butylcyclohexanol, a mixture of cis-4-tert-butylcyclohexanol and trans-4-tert-butylcyclohexanol, a topically acceptable salt of any of them; and a combination of any of them with a glycol for avoiding the sting of a poisonous organism that contain cnidocytes, in particular of jellyfish such as P. *noctiluca.* In particular, the effective amount for avoiding the sting of poisonous organism is an effective amount for inhibiting nematocysts discharge of the poisonous organism that contain cnidocytes.

It is also part of the invention, the use of a topical composition comprising an effective amount for avoiding the sting of a poisonous organism of an active ingredient selected from the group consisting of hydroxy acetophenone, a topically acceptable salt thereof, a combination of hydroxy acetophenone with a glycol, and a combination of a topically acceptable salt of hydroxy acetophenone with a glycol, together with one or more appropriate topical acceptable excipients or carriers In particular, the effective amount for avoiding the sting of poisonous organism is an effective amount for inhibiting nematocysts discharge of the poisonous organism that contain cnidocytes.

The use of the topical composition as defined above may further comprises combining the active ingredient or the combination with the glycol with a further active ingredient which is selected from the group consisting of cis-4-tert-butylcyclohexanol, trans-4-tert-butylcyclohexanol, a mixture of cis-4-tert-butylcyclohexanol and trans-4-tert-butylcyclohexanol, and a topically acceptable salt of any of them. The amount of this further active ingredient in the topical composition is an effective amount for avoiding the sting of a of poisonous organism. In particular, the effective amount for avoiding the sting of poisonous organism is an effective amount for inhibiting nematocysts discharge of the poisonous organism that contain cnidocytes.

The previous uses can also be formulated as a method for avoiding the sting of a poisonous organism that contain cnidocytes comprising the topical administration of topical composition as defined above, prior to contact with said organism.

The compositions may be in various topical forms such as solutions, emulsions (lotions, creams), gels, gel cream, and the like. In another particular embodiment of this use, the topical composition is in form of a solution. These solutions are advantageous because can be applied with less friction and mechanical effort.

In another particular embodiment of the use according to the invention, the topical composition comprises a solvent.

In a particular embodiment, the solvent is selected from water, ethanol, and mixtures thereof. The solution can be administered as drops or spray to the affected area.

The glycols in the composition may also act as solvents. In a particular embodiment, the amount of the glycol in the composition may vary from 0.5wt% to 99.5wt %, based on the total weight of the composition. The glycol used may be also a mixture of two or more glycols.

In a particular embodiment of the use according to the invention, the glycol and the further solvent are in a weight ratio of from 80:20 to 1:99. In a particular embodiment of the use according to the invention, the glycol and the further solvent are in a weight ratio of from 80:20 to 5:95.

In a particular embodiment of the use according to the invention, glycol and water are used in the topical composition in a weight ratio glycol/water of from 80:20 to 5:95. In another particular embodiment of the use according to the invention, glycol and water are used in the topical composition in a weight ratio of from 80:20 to 10:90. In another particular embodiment of the use according to the invention, glycol and water are used in the topical composition in a weight ratio of from 80:20 to 10:80. In a particular embodiment of the use according to the invention, glycol and water are used in the topical composition in a weight ratio of from 80:20 to 40:60. In another particular embodiment of the use according to the invention glycol and water are in a weight ratio of from 75:25 to 40:60. In another particular embodiment of the use according to the invention, glycol and water are in a weight ratio of from 60:40 to 40:60. In another particular embodiment of the use according to the invention, glycol and water are in a weight ratio of from 52:48 to 48:52. In another particular embodiment of the use according to the invention, glycol and water are in a weight ratio of 50:50. The glycol used may be also a mixture of two or more glycols. Preferably, the glycol used in these solutions is butylene glycol or propylene glycol. In another particular embodiment in combination with the embodiments above, the glycol is butylene glycol. In another particular embodiment in combination with the embodiments above, the glycol is propylene glycol.

In another particular embodiment of the use according to the invention, glycol and ethanol are used in the topical composition in a weight ratio glycol/ethanol of from 80:20 to 5:95. In another particular embodiment of the use according to the invention, glycol and ethanol are used in the topical composition in a weight ratio of from 80:20 to 10:90. In another particular embodiment of the use according to the invention, glycol and ethanol are used in the topical composition in a weight ratio of from 80:20 to 20:80. In another particular embodiment of the use according to the invention, glycol and ethanol are used in the topical composition in a weight ratio of from 80:20 to 40:60. In another particular embodiment of the use according to the invention, glycol and ethanol are in a weight ratio of from 75:25 to 40:60. In another particular embodiment of the use according to the invention, glycol and ethanol are in a weight ratio of from 60:40 to 40:60. In another particular embodiment of the use according to the invention, glycol and ethanol are in a weight ratio of from 52:48 to 48:52. In another particular embodiment of the use according to the invention, glycol and ethanol are in a weight ratio of 50:50. Preferably, the glycol used in these solutions is butylene glycol or propylene glycol. The glycol used may be also a mixture of two or more glycols. In a particular embodiment in combination with the embodiments above, the glycol is butylene glycol. In another particular embodiment in combination with the embodiments above, the glycol is propylene glycol.

In another particular embodiment of the use according to the invention, the glycol is pentylene glycol. In another particular embodiment the pentylene glycol is in an amount of 0.5-3 wt% based on the total weight of the composition.

In another particular embodiment of the use according to the invention, the topical composition comprises an amount of from 0.25 to 5wt% of any of the active ingredients present in the composition, wherein these active ingredients are any of hydroxy acetophenone, cis-4-tert-butylcyclohexanol, trans-4-tert-butylcyclohexanol, a mixture of cis-4-tert-butylcyclohexanol and trans-4-tert-butylcyclohexanol, or a topically acceptable salt of any of them, based on the total weight of the composition.

It is also part of the invention, the topical composition described above as product *per se,* i.e., a topical composition comprising an effective amount for avoiding the sting of poisonous organism of an active ingredient as defined above or mixture of active ingredients as defined above, together with one or more appropriate topical acceptable excipients or carriers. The topical composition may further comprise a glycol. The particular embodiments defined above for the use of the topical composition are also particular embodiments for the composition *per se.* In particular, the effective amount for avoiding the sting of poisonous organism is an effective amount for inhibiting nematocysts discharge of the poisonous organism that contain cnidocytes.

In a particular embodiment, the topical composition is that where hydroxy acetophenone is in free form. In another particular embodiment the topical composition is that comprising a combination of hydroxy acetophenone in free form with a glycol. In another particular embodiment, the topical composition further comprises 4-t-butylcyclohexanol (cis, trans or mixture) in free form.

The excipients of the topical composition may be cosmetically acceptable, pharmaceutically acceptable, or dermatological acceptable excipients.

In a particular embodiment, the topical composition is a sunscreen composition. In another particular embodiment, the sunscreen composition has a high sun protection factor (SPF) equal to or higher than 6, preferably, equal to or higher than 30, more preferably, equal to or higher than 50. Preferably, the sunscreen composition comprises physical, chemical filters, or combinations thereof, together with topically acceptable excipients or carriers appropriate for topical application to a human skin. The sunscreen composition may be in a galenic form such as emulsion (lotions, creams), gel, gel cream, solution, or spray.

In another particular embodiment of the topical composition (sunscreen composition), the hydroxy acetophenone or its topically acceptable salts, are in an amount comprised between 0.25-5 wt% based on the total weight of the composition. In another particular embodiment of the topical composition the hydroxy acetophenone or its topically acceptable salts, are in an amount comprised between 0.25-3 wt% based on the total weight of the composition. In another particular embodiment of the topical composition, the hydroxy acetophenone or the topically acceptable salt thereof is in an amount comprised between 0.5-2% wt% based on the total weight of the composition. In another particular embodiment of the topical composition, the hydroxy acetophenone or the topically acceptable salt thereof is in an amount comprised between 0.5-1.5% wt% based on the total weight of the composition. In another particular embodiment of the topical composition, the hydroxy acetophenone is in an amount comprised between 1.0-1.5 wt% based on the total weight of the composition.

In another particular embodiment, when a further active ingredient selected from the group consisting of cis-4-tert-butylcyclohexanol, trans-4-tert-butylcyclohexanol, a mixture of cis-4-tert-butylcyclohexanol and trans-4-tert-butylcyclohexanol, and a topically acceptable salt of any of them is present, is in an amount comprised between 0.25-5 wt% based on the total weight of the composition. In another particular embodiment of the topical composition, the further active ingredient or the topically acceptable salt thereof is in an amount comprised between 0.5-3% wt% based on the total weight of the composition. In another particular embodiment of the topical composition, the further active ingredient or the topically acceptable salt thereof is in an amount comprised between 0.5-2% wt% based on the total weight of the composition. In another particular embodiment of the topical composition, the further active ingredient or the topically acceptable salt thereof is in an amount comprised between 0.5-1.5% wt% based on the total weight of the composition. In another particular embodiment of the topical composition, the further active ingredient is in an amount comprised between 0.6-1.2 wt% based on the total weight of the composition.

In another particular embodiment, the topical composition is a sunscreen composition, and the glycol is in an amount equal to or below than 30 wt% based on the total weight of the composition.

In a particular embodiment, the topical composition comprises hydroxyacetophenone, butylene glycol, and water. In another particular embodiment, the topical composition comprises hydroxyacetophenone, and ethanol. In another particular embodiment, the topical composition comprises hydroxyacetophenone, ethanol, and water. In another particular embodiment, the topical composition comprises hydroxyacetophenone, propylene glycol, and ethanol. In another particular embodiment, the topical composition comprises hydroxyacetophenone, propylene glycol, ethanol, and water. In another particular embodiment, the topical composition comprises hydroxyacetophenone, butylene glycol, ethanol, and water. In any of these mixtures a second active ingredient may be present, in particular, 4-tert-butylcyclohexanol (cis, trans, or mixtures).

The preparation of topically acceptable salts of the hydroxy acetophenone can be carried out by methods known in the art. Generally, such salts are, for example, prepared by reacting the hydroxy acetophenone with a stoichiometric amount of the appropriate pharmaceutically acceptable base in a solvent such as water, an organic solvent, or a mixture of them.

The topical compositions of the present invention can be produced by conventional processes known in the art, such as for instance by mixing the different components of the composition, in any order.The appropriate excipients and/or carriers, and their amounts, can readily be determined by those skilled in the art according to the type of formulation being prepared.

Finally, it is considered also part of the invention, a topical composition comprising an effective amount for avoiding the sting of a poisonous organism of an active ingredient selected from the group consisting of cis-4-tert-butylcyclohexanol, trans-4-tert-butilcyclohexanol, a mixture of cis-4-tert-butylcyclohexanol and trans-4-tert-butilcyclohexanol, a topically acceptable salt of any of them, and a combination of any of them with a glycol, together with one or more appropriate topical acceptable excipients or carriers, wherein the topical composition is a preventive sunscreen composition. In particular, the effective amount for avoiding the sting of poisonous organism is an effective amount for inhibiting nematocysts discharge of the poisonous organism that contain cnidocytes.

In a particular embodiment, the topical composition comprises 4-tert-butylcyclohexanol (cis, trans, or mixtures), butyleneglycol, and ethanol. In another particular embodiment, the topical composition comprises 4-tert-butylcyclohexanol (cis, trans, or mixtures), butylene glycol, ethanol, and water. The particular embodiments for the topical compositions described above for other active ingredients are also particular embodiments for this topical composition.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

Jellyfish cultures: Jellyfish were cultivated by laboratory conditions in Loro Parque aquarium (Tenerife, Spain). The P. *noctiluca* adult individuals were breeding in kreisels tanks with a continuous water renewal, 33-35‰ salinity, 22°C temperature and light cycle of 12h light/12h dark. Individuals were daily fed with *Artemia* sp. nauplii, small pieces of the jellyfish *Aurelia* sp., eggs of *Merluccius* and *Parapenaeus longirostris.*

### Example 1: Evaluation of the inhibitor effect by chemical stimulation of nematocysts discharge. Tentacle Solution Assay (TSA).

Tentacles pieces of approximately 3 cm long were transferred to slides (76x26 cm). The samples were observed under the light microscope in order to ensure their integrity. Subsequently, tentacles were incubated on microwells with 3 ml of each solution for 5 minutes. In order to know the inhibitory effect, the neutral solutions were chemically stimulated by means of 15 µl of 5% acetic acid solution in distilled water (an activator solution *per se*)*.*

The nematocysts response was classified qualitatively in four categories in accordance with Pyo et al. (2016):
1. 0: no discharge was observed;
2. +: low discharge of nematocysts;
3. ++: medium discharge of nematocysts;
4. +++: high discharge of nematocysts.

The effect was classified in one of three categories:
1. Neutral effect solution: nematocysts were not activated after the incubation in the first solution but were activated by the subsequent chemical stimulation with 5% acetic acid solution;
2. Reductor effect solution: nematocysts were not activated after the incubation in the first solution, but some isolated nematocysts discharge was observed activated by the subsequent chemical stimulation with 5% acetic acid solution;
3. Inhibitory effect solution: nematocysts were not activated after the incubation in the first solution or by the subsequent chemical stimulation with 5% acetic acid solution.

**Table 1. Inhibitory efficacy on the nematocyst discharge in P. noctiluca.**

| **Tested solutions (wt%)** | **Inhibitory effect (TSA) using 5% acetic acid as chemical stimulator** | |
|---|---|---|
| | **Discharge** | **Effect** |
| Chemical stimulation application - 5% Acetic acid solution | +++ | Activator |
| 100% Seawater (control) | +++ | Neutral |
| 50% Butylene Glycol + 50% distilled water | ++ | Neutral |
| 1.5% 4-HAP in 48.5% distilled water + 50% butylene glycol | 0 | Inhibitor |
| 100% Butylene glycol | + | Reductor |
| 100% Propylene glycol | + | Reductor |
| 1.5% HAP + 48.5% ethanol + 50% propylene glycol | +/0 | Inhibitor |
| 100% Ethanol | ++ | Neutral/Reductor |
| 1.5% 4-HAP + 98.5% ethanol | 0 | Inhibitor |
| 1.5% 2-HAP + 98.5% ethanol | 0 | Inhibitor |
| 1.5% 3-HAP + 98.5% ethanol | 0 | Inhibitor |
| 1.2% trans 4-t-butylcyclohexanol + 1.8% pentylene glycol in 97% butylene glycol | 0 | Inhibitor |
| 0.6% trans 4-t-butlycyclohexanol + 0.9% pentylene glycol in a mixture of 48.5% butylene glycol + 50% ethanol | 0 | Inhibitor |
| 1.2% trans 4-t-butylcyclohexanol + 1.8% pentylene glycol in 97% ethanol | 0 | Inhibitor |
| 1.2% 4-tert-butylcyclohexanol cis/trans 1:1 in ethanol | + | Inhibitor |
| 0.6% 4-tert-butylcyclohexanol cis/trans 1:1 in ethanol | + | Inhibitor |

### Example 2: Natural stimulation of nematocyst discharge. Tentacle Skin Blood Agarose Assay (TSBAA).

To corroborate the irreversibly prevented nematocyst discharge of the reductor or inhibitory solutions in Method 3, the venom load following contact with the tentacle was evaluated by quantification of the hemolytic area in a modified protocol adapted from the TSBAA method published by Yanagihara et al. (2016) (Yanagihara et al., 2016). Briefly, an agarose gel preparation incorporating sheep red blood cells (SRBCs) (Thermo Fisher Scientific) was used covered by a thin tissue of pig intestine to simulate the effect of the human skin barrier.

A solution of 4% low melting point agarose (INVITROGEN ^{™} LMP Agarose) in phosphate buffered saline (PBS) (SIGMA) was prepared and cooled to room temperature for a few minutes. The SRBCs were centrifuged and resuspended in PBS until a final concentration of 2% was obtained. Equal volumes of both preparations were mixed and poured onto a glass surface with a mold (18x7.5 cm) and allowed to cool to room temperature. Once the agarose with SRBCs was set, rectangles were cut and placed on slides and kept at 4 °C in a humidification chamber (maximum 72 hours until use).

Pig small intestine tissue was washed in a 50 mM saline solution and then cut in rectangular sections. The sections were sterilized in a disinfecting solution consisting of 87% 110 mM saline, 10% ethanol (PANREAC, ethanol 96%) and 3% hydrogen peroxide for 3 hours, after which they were left in physiological serum overnight. The next day, the sections were stretched on a glass surface and allowed to dry for a few minutes in the open air. The pretreated sections of the pig small intestine were placed on the SRBC agarose rectangles.

Cutting tentacles of the same size is a complicated process due to the contraction and expansion of the tentacles after their manipulation. In order to control the sting area, we design plastic molds obtained through a 3D printer.

Tentacle pieces of approximately 3 cm long were incubated on reductor or inhibitory solutions (Method 3) for 5 minutes. Consecutively, the agarose rectangles were stung by a sting diameter of 57,35 mm² for 1 minute. A weight of 0,66 g was applied to ensure the contact between the tentacle and the skin. After the sting process, the plastic molds and the intestine sections were removed and the SRBC's agarose rectangles were stored in the humidification chamber at room temperature. After a period of 22 hours, images of the hemolytic areas were obtained with a camera. Hemolytic areas were calculated using the Fiji version of ImageJ software (J. Schindelin et al., Fiji: an open-source platform for biological-image analysis. Nature methods, 2012, vol. 9, pp. 676-682).

**Table 2. Inhibitory efficacy on the venom load to P. noctiluca.**

| **Tested solutions (wt%)** | **Venom load - Hemolytic area (%)** | **% Inhibitory** |
|---|---|---|
| 100% Seawater (control) | 60.77 ± 31.38 | 0 |
| 100% Butylene glycol | 26.54 ± 19.66 | 56.33 |
| 1.5% HAP in 48.5% water + 50% butylene glycol | 2.48 ±4 .54 | 95.92 |
| 1.2% trans 4-t-butylcyclohexanol + 1.8% pentylene glycol in 97% butylene glycol | 11.00±17.66 | 81.90 |
| 0.6% trans 4-t-butylcyclohexanol + 0.9% pentylene glycol in a mixture of 48.5% butylene glycol + 50% ethanol | 1.95±2.43 | 96.80 |

The results show that HAP significantly reduces the discharge of nematocysts. The results also show that 4-tert-butyl cyclohexanol significantly reduces the discharge of nematocysts.

### Citation List

### Patent Literature

- EP2380577A1
- EP2363135A1
- WO 98/53807A1

### Non Patent Literature

- A Ballesteros et al in "Differing Effects of Vinegar on Pelagia noctiluca (Cnidaria: scyphozoa) and Carybdea marsupialis (Cnidaria: Cubozoa) sting. Implications for First Aid Protocols", Toxins, 2021, vol. 13, p.509
- Salleo et al in J. exp. Biol., 1994, vol. 187, pp. 201-206
- A. Yanagihara, et al., "Experimental assays to assess the efficacy of vinegar and other topical first-aid approaches on cubozoan (Alatina alata) tentacle firing and venom toxicity" Toxins 2016, vol. 8, p. 19
- Pyo et al. "Modulation of jellyfish nematocyst discharges and management of human skin stings in Nemopilema nomurai and Carybdea mora." Toxicon 2016, vol.109, pp. 26-32
- J. Schindelin et al., Fiji: an open-source platform for biological-image analysis. Nature methods, 2012, vol. 9, pp. 676-682

## Claims

1. Use of an active ingredient selected from the group consisting of hydroxy acetophenone, a combination of hydroxy acetophenone with a glycol, and a combination of a topically acceptable salt of hydroxy acetophenone with a glycol, for avoiding the sting of a poisonous organism that contain cnidocytes.

2. Use according to claim 1, wherein the sting of poisonous organism is avoided by inhibiting nematocysts discharge of the poisonous organism that contain cnidocytes.

3. Use according to any of the claims 1-2, wherein the poisonous organism is a jellyfish.

4. Use according to any of the claims 1-3, wherein the jellyfish Is a *Pelagia noctiluca.*

5. Use according to any of the claims 1-4, wherein the hydroxy acetophenone is selected from the group consisting of 2-hdyroxyacetophenone, 3-hydroxyacetophenone, 4-hdyroxyacetophenone, and mixtures thereof.

6. Use according to any of the claims 1-5, wherein the glycol is selected from the group consisting of butylene glycol, propylene glycol, and mixtures thereof.

7. Use according to any of the claims 1-6, wherein the hydroxy acetophenone, or a combination of hydroxy acetophenone with a glycol forms part of a topical composition.

8. Use according to claim 7, wherein the topical composition further comprises a solvent selected from water, ethanol, and mixtures thereof.

9. Use according to any of the claims 1-8, wherein the glycol and the further solvent are in a weight ratio of from 80:20 to 5:95.

10. Use according to any of the claims 1-9, further comprising combining the active ingredient or the combination of the active ingredient with the glycol, with a second active ingredient which is selected from the group consisting of cis-4-tert-butylcyclohexanol, trans-4-tert-butilcyclohexanol, a mixture of cis-4-tert-butylcyclohexanol and trans-4-tert-butilcyclohexanol, and a topically acceptable salt of any of them.

11. Use of an active ingredient selected from the group consisting of cis-4-tert-butylcyclohexanol, trans-4-tert-butilcyclohexanol, mixtures of cis-4-tert-butylcyclohexanol and trans-4-tert-butilcyclohexanol, topically acceptable salts of any of them, and a combination of any of them with a glycol, for inhibiting nematocysts discharge of poisonous organism that contain cnidocytes.

12. A topical composition comprising an effective amount for avoiding the sting of a poisonous organism of an active ingredient or mixture of active ingredients as defined in any of the claims 1-10, together with one or more appropriate topical acceptable excipients or carriers.

13. The topical composition according to claim 12, which is a sunscreen composition.

14. The topical composition according to any of the claims 12-13, wherein the effective amount for inhibiting the nematocysts discharge of a poisonous organism comprises an amount from 0.25 to 5 wt% based on the total weight of the composition for each of the active ingredients present in the composition, wherein these ingredients are any of hydroxy acetophenone, cis-4-tert-butylcyclohexanol, trans-4-tert-butylcyclohexanol, a mixture of cis-4-tert-butylcyclohexanol and trans-4-tert-butylcyclohexanol, or a topically acceptable salt of any of them.

15. The topical composition according to claim 14, wherein the effective amount is an amount from 0.25 to 3 wt%.
